# EUROPEAN PATENT APPLICATION

(11) **EP 4 431 096 A1**
(43) Date of publication of application: **18.09.2024**
(21) Application number: 22892798.4
(22) Date of filing: 09.11.2022
(51) Int. Cl.: A61K 31/4433, A61K 9/20, A61K 9/30, A61K 9/32, A61K 9/36, A61K 47/32, A61K 47/36, A61K 47/38, A61P 35/00, A61P 43/00

(54) **PHARMACEUTICAL COMPOSITION HAVING EXCELLENT ELUTION PROPERTY**

(30) Priority: 10.11.2021 JP 2021183134
(71) Applicant: Daiichi Sankyo Company, Limited, Tokyo 103-8426 (JP)
(72) Inventor: WATANABE, Naho, Tokyo 103-8426 (JP); YAMAKOSE, Hiroshi, Tokyo 103-8426 (JP); TAKEMURA, Masami, Tokyo 103-8426 (JP)
(74) Representative: Marks & Clerk LLP
(86) International application number: PCT/JP2022/041656
(87) International publication number: WO 2023/085300

(57) **Abstract**

The present invention provides a pharmaceutical composition in which valemetostat or a pharmaceutically acceptable salt thereof, particularly valemetostat tosylate, has excellent dissolution properties. It was found that excellent dissolution properties can be achieved by combining valemetostat or a pharmaceutically acceptable salt thereof with one or more of croscarmellose sodium and sodium starch glycolate.

## Description

### Technical Field

The present invention relates to a pharmaceutical composition in which valemetostat, or a pharmaceutically acceptable salt thereof, has excellent dissolution properties.

### Background Art

The polycomb family negatively regulates gene expression through chromatin regulation via histone modifications. Enhancer of zeste homologue 1/2 (EZH1/2) is the active center of Polycomb repressive complex 2 (PRC2), which trimethylates histone H3K27. EZH1 and EZH2 compensate for each other's functions and maintain the intracellular epigenome. Inhibition of EZH2 leads to a reduction in the methylation level of H3K27 throughout the cell, but the effect is limited by the compensatory effect of EZH1. Simultaneous inhibition of EZH1 and EZH2 allows to more effectively eliminate methylation (Non Patent Literature 1). Abnormalities in PRC2 components have been shown to lead to cancer and abnormalities in stem cell function. In particular, accumulation of methylated H3K27me3 induced by genetic abnormalities or increased expression of EZH2 has been identified in many cancers, and research has been intensively pursued mainly on EZH2 as a new molecular target for cancer (Non Patent Literatures 2 and 3).

Valemetostat and pharmaceutically acceptable salts thereof are known as EZH1/2 dual inhibitors (Patent Literature 1).
Valemetostat is a compound represented by the following structural formula:

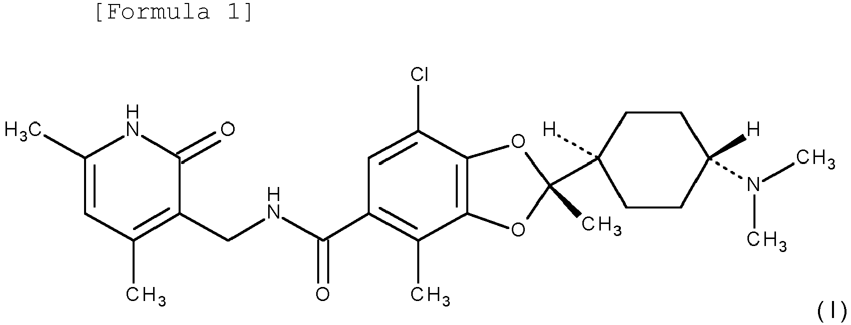

(hereinafter also referred to as the compound represented by formula (I)), and is known to have excellent antitumor activity.

In order for a drug to perform appropriately, the dissolution properties of the preparation in the body are important. When the preparation is orally administered, it is rapidly disintegrated and absorbed in the body. However, sufficient dissolution properties may not be obtained where the drug changes to a less soluble state such as to prevent disintegration and dispersion, which are necessary for rapid dissolution of the preparation. A method of adding silicates to improve the dissolution properties (Patent Literature 2) and a method of producing a disintegrable tablet core containing the drug (Patent Literature 3) are known.

### Citation List

### Patent Literature

Patent Literature 1: WO2015141616
Patent Literature 2: WO2006030826
Patent Literature 3: Japanese Laid-Open No. S62-123118 Non Patent Literature

Non Patent Literature 1: Shen, X et al., Mol Cell 2008; 32(4): 491-502.
Non Patent Literature 2: Sparmann A, van Lohuizen M., Nat Rev Cancer 2006; 6: 846.
Non Patent Literature 3: Lund, Adams, Copland., Leukemia 2014; 28(1): 44-9.

### Summary of Invention

### Technical Problem

The object of the present invention is to provide a pharmaceutical composition in which valemetostat or a pharmaceutically acceptable salt thereof, particularly valemetostat tosylate, has excellent dissolution properties. This is because the present inventors have found that, although valemetostat tosylate exhibits sufficient solubility under vigorous agitation, under slow agitation, as in the dissolution test used to evaluate the dissolution properties of a drug from an oral solid preparation, such as the paddle method of the dissolution test method in the Japanese Pharmacopoeia at 50 rotations per minute, the drug on the surface of the preparation changes to a translucent state and prevents the dissolution of the drug inside, resulting in very low dissolution, i.e., the dissolution rate expected from the solubility is not obtained. Since the effect of vigorous agitation cannot be expected in vivo, a pharmaceutical composition having sufficient dissolution properties in vivo is needed.

### Solution to Problem

The present inventors have conducted extensive studies to solve the problem. As a result, they have found that excellent dissolution properties can be achieved by combining valemetostat or a pharmaceutically acceptable salt thereof with one or more of croscarmellose sodium and sodium starch glycolate. Surprisingly, they have further found that the excellent dissolution properties are retained even after long-term storage in the case of film-coated tablets, and thereby completed the present invention.

The present invention relates to the following (1) to (10).
(1) A pharmaceutical composition comprising valemetostat or a pharmaceutically acceptable salt thereof, and one or more selected from croscarmellose sodium and sodium starch glycolate.
(2) The pharmaceutical composition according to (1), further comprising a water-insoluble excipient.
(3) The pharmaceutical composition according to (2), wherein the water-insoluble excipient is one or more selected from microcrystalline cellulose, partially pregelatinized starch, and pregelatinized starch.
(4) The pharmaceutical composition according to (2) or (3), wherein the water-insoluble excipient is microcrystalline cellulose.
(5) The pharmaceutical composition according to any one of (1) to (4), comprising 5 to 25% by mass of croscarmellose sodium relative to the total mass of the pharmaceutical composition.
(6) The pharmaceutical composition according to any one of (1) to (5), comprising 5 to 45% by mass of sodium starch glycolate relative to the total mass of the pharmaceutical composition.
(7) The pharmaceutical composition according to any one of (1) to (6), further comprising one or more lubricants.
(8) The pharmaceutical composition according to any one of (1) to (7), which is a tablet.
(9) The pharmaceutical composition according to (8), which is a film-coated tablet.
(10) The pharmaceutical composition according to (9), wherein the film-coating component is one or more selected from hypromellose, polyvinyl alcohol, and polyvinyl alcohol/acrylic acid/methyl methacrylate copolymer.

### Advantageous Effects of Invention

The pharmaceutical composition of the present invention allows rapid disintegration and dispersion of a pharmaceutical composition containing valemetostat or a pharmaceutically acceptable salt thereof, which changes to a less soluble state when in contact with a solution. Furthermore, making it into a film-coated tablet makes it possible to obtain a pharmaceutical composition that ensures rapid dissolution over a long period of time.

### Description of Embodiments

Valemetostat is a compound represented by formula (I) of the present invention, and is also referred to as (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide, valemetostat, or DS-3201a. Valemetostat can be produced, for example, according to the method described in Example 35 of WO2015/141616. WO2015/141616 is incorporated herein by reference in its entirety.

The pharmaceutically acceptable salt of valemetostat is most preferably (2R)-7-chloro-2-[trans-4-(dimethylamino)cyclohexyl]-N-[(4,6-dimethyl-2-oxo-1,2-dihydropyridin-3-yl)methyl]-2,4-dimethyl-1,3-benzodioxol-5-carboxamide p-toluenesulfonate, which is also referred to as valemetostat tosylate, or DS-3201b.

Valemetostat and valemetostat tosylate are sometimes collectively referred to as DS-3201.

The "croscarmellose sodium" used in the present invention may be any croscarmellose sodium that can be used as a pharmaceutical additive. Examples thereof include Ac-Di-Sol (registered trademark) (manufactured by DuPont), SOLUTAB (registered trademark) (manufactured by ROQUETTE), and Primellose (registered trademark) (manufactured by DFE Pharma), and it is preferably Primellose.

The blending ratio of croscarmellose sodium used in the pharmaceutical composition in the present invention is preferably 5 to 25% by mass relative to the total mass of the pharmaceutical composition. More preferably, it is 7 to 25% by mass, still more preferably 10 to 25% by mass, and most preferably 10 to 20% by mass.

The "sodium starch glycolate" used in the present invention may be any sodium starch glycolate that can be used as a pharmaceutical additive. Examples thereof include Primojel (registered trademark) (manufactured by DFE Pharma), GLYCOLYS (registered trademark) (manufactured by ROQUETTE), and Explotab (registered trademark) (manufactured by JRS Pharma), and it is preferably Explotab.

The blending ratio of sodium starch glycolate used in the pharmaceutical composition in the present invention is preferably 5 to 45% by mass relative to the total mass of the pharmaceutical composition. More preferably, it is 5 to 30% by mass.

Examples of the "excipient" used in the present invention include organic excipients such as sugar derivatives such as lactose, lactose hydrate, white sugar, glucose, mannitol or sorbitol; starch derivatives such as corn starch, granulated corn starch, potato starch, pregelatinized starch, partially pregelatinized starch, dextrin, wheat flour, wheat starch, wheat germ flour, rice powder, or rice starch; cellulose derivatives such as microcrystalline cellulose, silicified microcrystalline cellulose, microcrystalline cellulose/carmellose sodium, powdered cellulose, synthetic aluminum silicate/hydroxypropyl starch/crystalline cellulose, hydroxypropyl cellulose, cellulose acetate phthalate, carmellose sodium, or hydroxypropyl starch; acacia; dextran; or pullulan; inorganic excipients such as silicate derivatives such as light anhydrous silicic acid, synthetic aluminum silicate, natural aluminum silicate, calcium silicate, magnesium silicate, magnesium aluminosilicate, or magnesium aluminometasilicate; phosphates such as dibasic calcium phosphate, tribasic calcium phosphate, anhydrous dibasic calcium phosphate, fine granulated dibasic calcium phosphate, or monobasic calcium phosphate; carbonates such as calcium carbonate or precipitated calcium carbonate; and sulfates such as calcium sulfate; and others such as crospovidone, wheat germ oil, titanium dioxide, magnesium oxide, talc, dihydroxyaluminum aminoacetate, magnesium carbonate, sodium stearyl fumarate, or magnesium stearate. The blending ratio of the excipient is not particularly limited as long as it is in accordance with the present invention, and may be appropriately set, for example, to improve the dissolution properties of the pharmaceutical composition of the present invention.

In the present specification, the "water-insoluble excipient" included in the "excipient" means an excipient insoluble in water, and examples thereof include carmellose sodium, crospovidone, magnesium aluminosilicate, calcium silicate, magnesium silicate, light anhydrous silicic acid, microcrystalline cellulose, silicified microcrystalline cellulose, microcrystalline cellulose/carmellose sodium, powdered cellulose, pregelatinized starch, partially pregelatinized starch, synthetic aluminum silicate, synthetic aluminum silicate/hydroxypropyl starch/crystalline cellulose, wheat flour, wheat starch, wheat germ flour, wheat germ oil, rice powder, rice starch, cellulose acetate phthalate, titanium dioxide, magnesium oxide, dihydroxyaluminum aminoacetate, tribasic calcium phosphate, talc, calcium carbonate, magnesium carbonate, precipitated calcium carbonate, natural aluminum silicate, corn starch, granulated corn starch, potato starch, hydroxypropyl starch, anhydrous dibasic calcium phosphate, fine granulated dibasic calcium phosphate, sodium stearyl fumarate, or magnesium stearate. Preferably, it is microcrystalline cellulose, partially pregelatinized starch, or pregelatinized starch, and more preferably, microcrystalline cellulose.

The total blending ratio of croscarmellose sodium, sodium starch glycolate, and the water-insoluble excipient used in the present invention in the pharmaceutical composition may be 10 to 90% by mass, 20 to 80% by mass, 30 to 70% by mass, or 45 to 70% by mass relative to the total mass of the pharmaceutical composition.

The "microcrystalline cellulose" used in the present invention may be any microcrystalline cellulose that can be used as a pharmaceutical additive, and can be used regardless of particle size or bulk density. Examples thereof include VIVAPUR (registered trademark) (manufactured by JRS Pharma), Ceolus (registered trademark) PH series (manufactured by Asahi Kasei), Ceolus (registered trademark) UF series (manufactured by Asahi Kasei), and Ceolus (registered trademark) KG series (manufactured by Asahi Kasei), and it is preferably Ceolus PH series, Ceolus UF series, or Ceolus KG series.

The blending ratio of microcrystalline cellulose used in the present invention in the pharmaceutical composition is preferably 1 to 60% by mass relative to the total mass of the pharmaceutical composition. More preferably, it is 19 to 55% by mass.

The "partially pregelatinized starch" used in the present invention may be any partially pregelatinized starch that can be used as a pharmaceutical additive. Examples thereof include Starch 1500G manufactured by Colorcon Japan and PCS (registered trademark) PC-10 manufactured by Asahi Kasei, and it is preferably Starch 1500G manufactured by Colorcon Japan.

The blending ratio of partially pregelatinized starch used in the present invention in the pharmaceutical composition is preferably 1 to 70% by mass relative to the total mass of the pharmaceutical composition. More preferably, it is 1 to 40% by mass.

The "pregelatinized starch" used in the present invention may be any pregelatinized starch that can be used as a pharmaceutical additive. Examples thereof include Starch 1500G manufactured by Colorcon Japan and SWELSTAR PD-1 manufactured by Asahi Kasei, and it is preferably Starch 1500G manufactured by Colorcon Japan.

The blending ratio of pregelatinized starch used in the present invention in the pharmaceutical composition is preferably 1 to 70% by mass relative to the total mass of the pharmaceutical composition. More preferably, it is 1 to 40% by mass.

In addition to the above components, the pharmaceutical composition of the present invention can further contain, as necessary, pharmaceutically acceptable additives such as lubricants, binders, emulsifiers, stabilizers, flavoring agents, coloring agents, disintegrants and/or preservatives as appropriate.

Examples of the "lubricant" used in the present invention include stearic acid; metallic stearates such as calcium stearate or magnesium stearate; talc; colloidal silica; waxes such as beeswax or spermaceti; boric acid; adipic acid; sulfates such as sodium sulfate; glycol; fumaric acid; sodium stearyl fumarate; sucrose esters of fatty acids; sodium benzoate; D,L-leucine; lauryl sulfates such as sodium lauryl sulfate or magnesium lauryl sulfate; silicates such as silicic anhydride or silicic acid hydrate; and starch derivatives. Preferably, it is magnesium stearate or sodium stearyl fumarate.

Examples of the "binder" used in the present invention include hydroxypropyl cellulose, hypromellose, polyvinylpyrrolidone, polyethylene glycol, or compounds similar to the excipients mentioned above. Preferably, it is hydroxypropyl cellulose.

Examples of the "emulsifier" used in the present invention include colloidal clays such as bentonite or veegum; metal hydroxides such as magnesium hydroxide or aluminum hydroxide; anionic surfactants such as sodium lauryl sulfate or calcium stearate; cationic surfactants such as benzalkonium chloride; and nonionic surfactants such as polyoxyethylene alkyl ethers, polyoxyethylene sorbitan fatty acid esters or sucrose esters of fatty acids.

Examples of the "stabilizer" used in the present invention include p-hydroxybenzoate esters such as methylparaben or propylparaben; alcohols such as chlorobutanol, benzyl alcohol or phenylethyl alcohol; benzalkonium chloride; phenols such as phenol or cresol; thimerosal; dehydroacetic acid; and sorbic acid.

Examples of the "flavoring agent" used in the present invention include sweeteners such as saccharin sodium or aspartame; acidulants such as citric acid, malic acid, or tartaric acid; and flavors such as menthol, lemon, or orange.

Examples of the "disintegrant" used in the present invention include cellulose derivatives such as low-substituted hydroxypropyl cellulose, carboxymethyl cellulose, carboxymethyl cellulose calcium, or sodium carboxymethyl cellulose; cross-linked polyvinylpyrrolidone; and chemically modified starches and celluloses such as carboxymethyl starch or sodium carboxymethyl starch. The components used as excipients in the present invention can also be used as disintegrants.

The blending amount of valemetostat or a pharmaceutically acceptable salt thereof in the pharmaceutical composition is not particularly limited, but for example, as valemetostat tosylate, 10 to 75% by mass (7 to 55% by mass as valemetostat), or 25 to 60% by mass (20 to 45% by mass as valemetostat) may be blended relative to the mass of the uncoated tablet before coating.

In the present invention, the blending amount of the additives in the pharmaceutical composition is not particularly limited, but for example, 0.5 to 10% by mass (preferably, 0.5 to 5% by mass) of lubricants and 0.1 to 10% by mass (preferably, 0.5 to 5% by mass) of binders may be blended relative to the total mass of the pharmaceutical composition.

The pharmaceutical composition of the present invention can be made into a solid preparation, such as a tablet (including a sublingual tablet and an orally disintegrating tablet), a capsule (including a soft capsule and a microcapsule), a granule, a fine granule, a powder, a pill, a chewable tablet or a lozenge. Preferably, it is a powder, a fine granule, a granule, a capsule, or a tablet, and more preferably a tablet.

The tablet, which is one aspect of the present invention, is preferably film-coated to make it a film-coated tablet. The film-coated tablet according to the present invention can have excellent dissolution properties and excellent storage stability.

The coating is performed, for example, using a coating pan, and examples of film-coating agents include sugar-coating agents, water-soluble film-coating agents, enteric film-coating agents, and sustained-release film-coating agents.

As a sugar-coating agent, white soft sugar is used, and furthermore, one or more selected from talc, precipitated calcium carbonate, calcium phosphate, calcium sulfate, gelatin, gum arabic, polyvinylpyrrolidone, and pullulan can also be used in combination.

Examples of water-soluble film-coating agents include cellulose derivatives such as hydroxypropyl cellulose, hypromellose, hydroxyethyl cellulose, hydroxypropyl cellulose, methyl hydroxyethyl cellulose or sodium carboxymethyl cellulose; synthetic polymers such as polyvinyl acetal diethylaminoacetate, aminoalkyl methacrylate copolymer, polyvinyl alcohol, polyvinyl alcohol/acrylic acid/methyl methacrylate copolymer or polyvinylpyrrolidone; and polysaccharides such as pullulan.

Examples of enteric film-coating agents include cellulose derivatives such as hydroxypropyl methylcellulose phthalate, hydroxypropyl methylcellulose acetate succinate, carboxymethyl ethyl cellulose, or cellulose acetate phthalate; acrylic acid derivatives such as (meth)acrylic acid copolymer L, (meth)acrylic acid copolymer LD, or (meth)acrylic acid copolymer S; polyvinyl alcohol; polyvinyl alcohol/acrylic acid/methyl methacrylate copolymer; and natural products such as shellac.

Examples of sustained-release film-coating agents include cellulose derivatives such as ethyl cellulose; and acrylic acid derivatives such as aminoalkyl methacrylate copolymer RS or ethyl acrylate/methyl methacrylate copolymer emulsion.

The preferable film-coating agent in the present invention is a water-soluble film-coating agent. More preferably, it is one or more film-coating agent selected from hypromellose, polyvinyl alcohol, and polyvinyl alcohol/acrylic acid/methyl methacrylate copolymer.

Two or more film-coating agents may be used by mixing them in appropriate proportions. In addition, if necessary, pharmaceutically acceptable plasticizers, excipients, lubricants, masking agents, coloring agents, and/or preservatives can be contained as appropriate.

The type of plasticizer that can be added to the film-coating agent of the present invention is not particularly limited and can be appropriately selected by those skilled in the art. Examples of plasticizers include propylene glycol, polyethylene glycol, polypropylene glycol, glycerin and sorbitol, glycerol triacetate, hydroxypropyl cellulose, vinyl acetate/vinylpyrrolidone copolymer, polyvinyl alcohol/acrylic acid/methyl methacrylate copolymer, diethyl phthalate and triethyl citrate, lauric acid, sucrose, dextrose, sorbitol, triacetin, acetyl triethyl citrate, triethyl citrate, tributyl citrate, or acetyl tributyl citrate.

Examples of masking agents that can be added to the film-coating agent of the present invention include titanium dioxide.

Other components, such as talc, can be added to the film-coating agent of the present invention.

Examples of coloring agents that can be used in the present invention include red ferric oxide, yellow ferric oxide, black iron oxide, titanium dioxide, Blue No. 1 (Brilliant Blue FCF), Blue No. 2 (Indigo carmine), Red No. 3 (Erythrosine), Yellow No. 4 (Tartrazine), and Yellow No. 5 (Sunset Yellow FCF). Preferably, it is red ferric oxide, yellow ferric oxide, or black iron oxide, and more preferably red ferric oxide or yellow ferric oxide.

Examples of the preservative that can be used in the present invention include parabens.

Other aspects of the present invention include a capsule.

The capsule of the present invention can contain croscarmellose sodium. Examples of croscarmellose sodium that can be used include Primellose (registered trademark) (DFE Pharma).

The capsule of the present invention can contain one or more lubricants. As a lubricant, lubricants commonly used in oral solid preparations can be used if excellent dissolution properties are exhibited, but sodium stearyl fumarate is preferably used.

The capsule of the present invention can contain partially pregelatinized starch. Examples of the partially pregelatinized starch that can be used include Starch 1500G manufactured by Colorcon Japan and PCS (registered trademark) PC-10 manufactured by Asahi Kasei.

A fluidizing agent can be added to the capsule of the present invention for the purpose of improving the fluidity of the mixed powder or improving the disintegration and dispersion abilities. Examples of the fluidizing agent include light anhydrous silicic acid.

The solid preparation of the present invention can be obtained as a powder, granule, surface-coated granule, capsule, tablet and surface-coated tablet by sequentially applying the following steps to a powder of the active ingredient valemetostat or a pharmaceutically acceptable salt thereof:
(1) adding croscarmellose sodium, sodium starch glycolate, excipients, disintegrants, and the like, as well as auxiliary agents (lubricant, etc.) necessary for formulation, and
(2) directly tableting the resulting mixture to obtain tablets or surface-coated tablets.
(3) Alternatively, making the resulting product from step (1) into a granular powder by dry granulation or wet granulation and adding other components as appropriate,
(4) a capsule filling step in which the resulting granular powder is compressed and filled by a capsule filling machine,
(5) or a tableting step in which the resulting granular powder is compressed by a tablet press, and
(6) if necessary, a coating step in which the surface of the obtained granular powder, granules, or tablets is coated.

Examples of the methods for producing a solid preparation include: (1) a direct tableting method, in which the active ingredient and the additives are mixed and compacted directly in a tablet press; (2) a semi-direct tableting method, in which the additives are made into granules, mixed with the drug and compacted; (3) a dry granulation method, in which the active ingredient and the additives are granulated together, partially separately or completely separately as granules by a dry method, and then the granules and lubricants are mixed as appropriate, and compacted; and (4) a wet granulation method, in which the active ingredient and the additives are granulated together or partially separately as granules by a wet method, and then a lubricant and the like are added thereto, followed by compaction. As a granulation method, means such as fluidized bed granulation, high-shear granulation, and melting granulation can be used. The production method in the present invention is preferably a direct tableting method, or a method in which the active ingredient and the additives are granulated as granules by a dry or a wet method, and then a lubricant and the like are added as needed, followed by compaction. Note that the lubricant may be added prior to granulation.

A preferable method for producing the tablet, which is an aspect of the present invention, will be further described.

After pulverizing the active ingredient valemetostat or a pharmaceutically acceptable salt thereof and adjusting its particle size, one or more of croscarmellose sodium and sodium starch glycolate, as well as one or more of an excipient, a binder, a disintegrant, and a lubricant are granulated by a dry method. The granules are then tableted in a tablet press to obtain tablets.

As another preferable aspect, after pulverizing the active ingredient valemetostat or a pharmaceutically acceptable salt thereof and adjusting its particle size, one or more of croscarmellose sodium and sodium starch glycolate, an excipient, a binder, and/or a disintegrant are granulated as granules by a wet method. Then, after adding a lubricant and further mixing, tablets are obtained by tableting in a tablet press.

Although it is difficult to generalize about the compression pressure in tablet production, as it is affected by tablet size, the tableting pressure in the tablet production of the present invention may be set in the range of 6 kN to 27 kN, or in the range of 12 kN to 24 kN for tablets of about 420 mg/tab. If necessary, the hardness and density can be specified to ensure quality.

By way of example, the stability test of the present specification can be performed by storing a package of the pharmaceutical composition packaged in a plastic bottle or a package of a FTP sheet containing the pharmaceutical composition as it is or sealed in an aluminum bag at 40°C/75%RH for 1, 3, or 6 months.

As for the stability test conditions, in addition to 40°C/75%RH, the evaluation can also be conducted over a short period of time. For example, the evaluation can be done for one week at 60°C or one week under open conditions at 40°C/75%RH. Short-term evaluation can be used if the dissolution of the pharmaceutical composition after storage at these temperatures/humidity and storage period is shown to be consistently lower than the dissolution after storage at 40°C/75% RH for 3 or 6 months.

The dose of valemetostat or the pharmaceutically acceptable salt thereof formulated in the present invention can vary depending on various conditions such as the activity of the drug, patient's symptoms, age or weight. The dose for oral administration is usually 25 mg (as valemetostat) per day for adults as the lower limit, and 500 mg (as valemetostat) can be administered as the upper limit. Preferably, it is 200 to 250 mg (as valemetostat) per day.

### Examples

Next, the present invention is further described in detail with reference to Examples and the like, but the following Examples are for the purpose of describing the present invention and should not be interpreted as limiting the present invention to these Examples.

### (Comparative Example 1)

Approximately 270 mg of valemetostat tosylate was filled into a die cavity with a diameter of 10 mm and compacted at a pressure of 1 metric ton. The molded product was not discharged from the die.

### (Comparative Example 2)

Valemetostat tosylate, light anhydrous silicic acid, and sodium stearyl fumarate were mixed in a mortar in the ratios shown in Table 1-1 to obtain a mixed powder. According to the ratios shown in Table 1-1, the mixed powder, pregelatinized starch, and low-substituted hydroxypropyl cellulose were mixed in a mortar for 2 minutes, and then sieved through a screen with an aperture of 500 µm to obtain a sieved powder. 500 mg of the sieved powder was filled into a die with a diameter of 15 mm, compacted at a pressure of 20 kN, and then crushed in a tablet grinder to obtain granules. 320 mg of the granules were filled into a die with a diameter of 9 mm and compacted at a pressure of 10 kN to obtain tablets.

### (Comparative Example 3)

Tablets were obtained in the ratios shown in Table 1-1, using the method according to Comparative Example 2.

### (Comparative Example 4)

Tablets were obtained in the ratios shown in Table 1-1, using the method according to Comparative Example 2.

### (Comparative Example 5)

Tablets were obtained in the ratios shown in Table 1-1, using the method according to Comparative Example 2.

### (Comparative Example 6)

Each component was weighed in the ratios shown in Table 1-1, mixed in a mortar for 2 minutes, and then sieved through a screen with an aperture of 500 µm to obtain a mixed powder. Granules and tablets were obtained using the method according to Comparative Example 2.

### (Example 1)

Valemetostat tosylate, sodium stearyl fumarate, and light anhydrous silicic acid were weighed in the ratios shown in Table 1-2 and mixed in a mortar for 3 minutes to obtain a mixed powder. 500 mg of the mixed powder was filled into a die with a diameter of 15 mm, compacted at a pressure of 20 kN, and then crushed in a tablet grinder to obtain granules. The granules and croscarmellose sodium were weighed in the ratios shown in Table 1-2 and then mixed for 30 seconds to obtain granules for tableting. 191.8 mg of the granules for tableting were filled into a die with a diameter of 8 mm and compacted at a pressure of 13 kN to obtain tablets.

### (Example 2)

Granules were obtained using the method according to Example 1. The granules, croscarmellose sodium, and sodium starch glycolate were each mixed in the ratios shown in Table 1-2 to produce granules for tableting. 239.8 mg of the granules for tableting were filled into a die with a diameter of 9 mm and compacted at a pressure of 14 kN to obtain tablets.

### (Example 3)

Tablets were obtained in the ratios shown in Table 1-2, using the method according to Comparative Example 2.

### (Example 4)

Tablets were obtained in the ratios shown in Table 1-2, using the method according to Comparative Example 2. However, the compression pressure was set at 14 kN.

### (Example 5)

Tablets were obtained in the ratios shown in Table 1-2, using the method according to Comparative Example 6.

### (Example 6)

Valemetostat tosylate, light anhydrous silicic acid, and sodium stearyl fumarate were weighed in the ratios shown in Table 1-2, mixed in a plastic bag for one minute, and then sieved through a screen with an aperture of 500 µm to obtain a mixed powder A. The mixed powder A was compressed using a roller compactor (TF-mini, manufactured by FREUND CORPORATION) at a roll pressure of 7 MPa and a roll rotation speed of 2.5 revolutions per minute, and then crushed using a Co-mil (QC-197, manufactured by Quadro) with a screen with a hole diameter of 0.813 mm to obtain granules A.

Microcrystalline cellulose and croscarmellose sodium were weighed in the ratios shown in Table 1-2 and mixed with a V-blender for 10 minutes to obtain a mixed powder B. The mixed powder B was compressed using a roller compactor (TF-mini, manufactured by FREUND CORPORATION) at a roll pressure of 7 MPa and a roll rotation speed of 2.5 revolutions per minute, and then crushed using a Co-mil (QC-197, manufactured by Quadro) with a screen with a hole diameter of 1.575 mm to obtain granules B. The fraction of the granules B that passed through a screen with an aperture of 850 µm and remained on a screen with an aperture of 150 µm was designated as granules B'.

The granules A and the granules B' were mixed with a V-blender for 10 minutes to obtain granules for tableting. 450 mg of the granules for tableting were filled into a die with a major diameter of 14 mm and a minor diameter of 6.5 mm and compacted at a pressure of 15 kN to obtain tablets.

### (Example 7)

Each component was weighed in the ratios shown in Table 1-2, mixed with a V-blender for 10 minutes, and then sieved to obtain a mixed powder. Using a tablet press (Vela, manufactured by Kikusui Seisakusho), 100 to 200 mg of the mixed powder was filled into a die with a diameter of 9.5 mm and compacted with a flat punch at a pressure of 8 to 10 kN to obtain slugs. The slugs were crushed using a Co-mil (QC-197, manufactured by Quadro) with a screen with a hole diameter of 0.991 mm to obtain granules. 231 mg of the granules were filled into a die with a diameter of 8.5 mm and compacted at a pressure of 14 kN to obtain tablets.

### (Example 8)

Tablets were obtained in the ratios shown in Table 1-2, using the method according to Comparative Example 2.

### (Example 9)

Granules were produced using the method according to Example 1. The granules and sodium starch glycolate were each mixed in the ratios shown in Table 1-3 to obtain granules for tableting. 264.8 mg of the granules for tableting were filled into a die with a diameter of 9 mm and compacted at a pressure of 11 kN to obtain tablets.

### (Example 10)

Tablets were obtained in the ratios shown in Table 1-3, using the method according to Comparative Example 6. The compression pressure was set at 7 kN.

### (Example 11)

Tablets were obtained in the ratios shown in Table 1-3, using the method according to Comparative Example 6.

### (Example 12)

Each component was weighed in the ratios shown in Table 1-3, mixed with a high-shear granulator to obtain a mixed powder. The mixed powder was compressed using a roller compactor (TF-mini, manufactured by FREUND CORPORATION) at a roll pressure of 5.5 MPa and a roll rotation speed of 2.5 revolutions per minute, and then crushed using a Power Mill (P-02S, manufactured by Dalton) with a screen with a hole diameter of 1 mm to obtain granules. Using a tablet press (Virgo01, manufactured by Kikusui Seisakusho), 420 mg of the granules were filled into a die with a major diameter of 14 mm and a minor diameter of 6.5 mm and compacted at a pressure of 18 kN to obtain uncoated tablets. Using a coating machine (HIGH-COATER lab, manufactured by FREUND CORPORATION), approximately 20 mg of the film-coating agent was film-coated on each tablet to obtain film-coated tablets. The amount of film-coating agent per tablet was determined from the mass difference of 10 or 20 tablets before and after film-coating. The same applies to the following Examples.

**[Table 1-1]**

| Component | Blending Amount (mg/tab) | | | | | |
|---|---|---|---|---|---|---|
| | Comparative Example 1 | Comparative Example 2 | Comparative Example 3 | Comparative Example 4 | Comparative Example 5 | Comparative Example 6 |
| Valemetostat Tosylate (As Valemetostat) | 270 (200) | 135.3 (100) | 135.3 (100) | 135.3 (100) | 135.3 (100) | 135.3 (100) |
| Low-Substituted Hydroxypropyl Cellulose (LH11, Shin-Etsu Chemical) | - | 31.5 | - | - | - | - |
| Carmellose Calcium (E.C.G-505, GOTOKU CHEMICAL COMPANY) | - | - | 31.5 | - | - | - |
| Crospovidone (Kollidon CL, BASF) | - | - | - | 31.5 | - | 32 |
| Carmellose (NS300, GOTOKU CHEMICAL COMPANY) | - | - | - | - | 31.5 | - |
| Croscarmellose Sodium (Primellose, DFE Pharma) | - | - | - | - | - | - |
| Sodium Starch Glycolate (Explotab, JRS Pharma) | - | - | - | - | - | - |
| Pregelatinized Starch (Starch 1500G, Colorcon Japan) | - | 128.7 | 128.7 | 128.7 | 128.7 | - |
| Partially Pregelatinized Starch (PCS PC-10, Asahi Kasei) | - | - | - | - | - | - |
| Microcrystalline Cellulose (Ceolus PH-301, Asahi Kasei) | - | - | - | - | - | 128.2 |
| Lactose (Dilactose S, FREUND CORPORATION) | - | - | - | - | - | - |
| Mannitol (Mannogem EZ, SPI Pharm) | - | - | - | - | - | - |
| Light Anhydrous Silicic Acid (Aerosil 200, NIPPON AEROSIL) | - | 7 | 7 | 7 | 7 | 7 |
| Light Anhydrous Silicic Acid (SYLYSIA 320TP, FUJI SILYSIA CHEMICAL) | - | - | - | - | - | - |
| Sodium Stearyl Fumarate (Pruv, JRS Pharma) | - | 17.5 | 17.5 | 17.5 | 17.5 | 17.5 |
| Magnesium Stearate (Spec Gx) | - | - | - | - | - | - |
| Uncoated Tablet Total | 270 | 320 | 320 | 320 | 320 | 320 |

| | | | | | | |
|---|---|---|---|---|---|---|
| "-" indicates that the component is not blended | | | | | | |

**[Table 1-2]**

| Component | Blending Amount (mg/tab) | | | | | | | |
|---|---|---|---|---|---|---|---|---|
| | Example 1 | Example 2 | Example 3 | Example 4 | Example 5 | Example 6 | Example 7 | Example 8 |
| Valemetostat Tosylate (As Valemetostat) | 135.3 (100) | 135.3 (100) | 135.3 (100) | 135.3 (100) | 135.3 (100) | 135.3 (100) | 135.3 (100) | 135.3 (100) |
| Croscarmellose Sodium (Primellose, DFE Pharma) | 32 | 48 | 31.5 | 64 | 16 | 31.5 | 33 | 31.5 |
| Sodium Starch Glycolate (Explotab, JRS Pharma) | - | 32 | - | - | - | - | - | - |
| Pregelatinized Starch (Starch 1500G, Colorcon Japan) | - | - | 128.7 | - | - | - | - | - |
| Partially Pregelatinized Starch (PCS PC-10, Asahi Kasei) | - | - | - | 96.2 | - | - | - | - |
| Microcrystalline Cellulose (Ceolus PH-301 , Asahi Kasei) | - | - | - | - | 144.2 | 258.7 | 45.1 | 128.7 |
| Light Anhydrous Silicic Acid (Aerosil 200, NIPPON AEROSIL) | 7 | 7 | 7 | 7 | 7 | 7 | 5.031 | 7 |
| Sodium Stearyl Fumarate (Pruv, JRS Pharma) | 17.5 | 17.5 | 17.5 | 17.5 | 17.5 | 17.5 | 12.58 | 17.5 |
| Uncoated Tablet Total | 191.8 | 239.8 | 320 | 320 | 320 | 450 | 231 | 320 |

| | | | | | | | | |
|---|---|---|---|---|---|---|---|---|
| "-" indicates that the component is not blended | | | | | | | | |

**[Table 1-3]**

| Component | Blending Amount (mg/tab) | | | |
|---|---|---|---|---|
| | Example 9 | Example 10 | Example 11 | Example 12 |
| Valemetostat Tosylate (As Valemetostat) | 135.3 (100) | 135.3 (100) | 135.3 (100) | 135.3 (100) |
| Croscarmellose Sodium (Primellose, DFE Pharma) | - | - | - | 63 |
| Sodium Starch Glycolate (Explotab, JRS Pharma) | 105 | 96 | 32 | 42 |
| Pregelatinized Starch (Starch 1500G, Colorcon Japan) | - | - | - | - |
| Partially Pregelatinized Starch (PCS PC-10, Asahi Kasei) | - | 64.2 | - | - |
| Microcrystalline Cellulose (Ceolus PH-301 , Asahi Kasei) | - | - | 128.2 | 167.1 |
| Light Anhydrous Silicic Acid (Aerosil 200, NIPPON AEROSIL) | 7 | 7 | 7 | - |
| Light Anhydrous Silicic Acid (SYLYSIA 320TP, FUJI SILYSIA CHEMICAL) | - | - | - | 6.3 |
| Sodium Stearyl Fumarate (Pruv, JRS Pharma) | 17.5 | 17.5 | 17.5 | - |
| Magnesium Stearate (Spec Gx) | - | - | - | 6.3 |
| Uncoated Tablet Total | 264.8 | 320 | 320 | 420 |
| Pre-mix Film-Coating Agent OPADRY (Colorcon Japan) | - | - | - | 21 |
| Film-Coated Tablet Total | - | - | - | 441 |

| | | | | |
|---|---|---|---|---|
| "-" indicates that the component is not blended | | | | |

### (Example 13)

Uncoated tablet components were weighed in the ratios shown in Table 2, and uncoated tablets were obtained using the method according to Example 12. Using a coating machine (HIGH-COATER lab, manufactured by FREUND CORPORATION), approximately 20 mg of the film-coating agent was film-coated on each tablet to obtain film-coated tablets.

### (Example 14)

Uncoated tablet components were weighed in the ratios shown in Table 2, and mixed with a V-blender for 10 minutes to obtain a mixed powder. The mixed powder was compacted using a roller compactor (TF-mini, manufactured by FREUND CORPORATION) at a pressure of 7 MPa and a roll rotation speed of 2.5 revolutions per minute to obtain a granulated material. The granulated material was crushed using a Co-mil (QC-U-5, manufactured by Quadro) with a screen with a hole diameter of 0.991 mm to obtain granules for tableting.

320 mg of the granules for tableting were filled into a die with a diameter of 9 mm and compacted at a pressure of 10 kN to obtain uncoated tablets. Using a coating machine (HIGH-COATER FZ, manufactured by FREUND CORPORATION), approximately 10 mg of the film-coating agent was film-coated on each tablet to obtain film-coated tablets. For Example 14, the composition is also listed in Table 4 for reference.

### (Example 15)

Uncoated tablet components were weighed in the ratios shown in Table 2, and mixed in a mortar to obtain a mixed powder. 500 mg of the mixed powder was filled into a die with a diameter of 15 mm, compacted at a pressure of 20 kN, and then crushed in a tablet grinder to obtain granules. 420 mg of the granules were filled into a die with a major diameter of 14 mm and a minor diameter of 6.5 mm and compacted at a pressure of 11 kN to obtain uncoated tablets. Using a coating machine (PRC-GTX mini, manufactured by Powrex), approximately 20 mg of the film-coating agent was film-coated on each tablet to obtain film-coated tablets.

### (Example 16)

Uncoated tablet components were weighed in the ratios shown in Table 2, and uncoated tablets were obtained using the method according to Example 12.

The film-coating components were weighed in the ratios shown in Table 2, and dissolved and suspended in water to obtain a film-coating solution. Using a coating machine (PRC-GTX mini, manufactured by Powrex), approximately 26 mg of the film-coating agent was film-coated on each tablet to obtain film-coated tablets.

### (Example 17)

Uncoated tablet components were weighed in the ratios shown in Table 2, and uncoated tablets were obtained using the method according to Example 12. However, a screen with a hole diameter of 2 mm was used for the Power Mill.

The film-coating components were weighed in the ratios shown in Table 2, and dissolved and suspended in water to obtain a film-coating solution. Using a coating machine (HIGH-COATER lab, manufactured by FREUND CORPORATION), approximately 24 mg of the film-coating agent was film-coated on each tablet to obtain film-coated tablets.

### (Example 18)

Uncoated tablet components were weighed in the ratios shown in Table 2, and mixed with a high-shear granulator for 10 minutes to obtain a mixed powder. Using a tablet press (Virgo01, manufactured by Kikusui Seisakusho), 300 mg of the mixed powder was filled into a die with a diameter of 15 mm, compacted at a pressure of 20 kN, and then crushed using a Power Mill (P-02S, manufactured by Dalton) with a screen with a hole diameter of 1 mm to obtain granules. Using a tablet press (Virgo01, manufactured by Kikusui Seisakusho), 420 mg of the granules were filled into a die with a major diameter of 14 mm and a minor diameter of 6.5 mm and compacted at a pressure of 19 kN to obtain uncoated tablets.

Using a coating machine (HIGH-COATER lab, manufactured by FREUND CORPORATION), approximately 21 mg of the film-coating agent was film-coated on each tablet to obtain film-coated tablets.

### (Example 19)

Uncoated tablet components were weighed in the ratios shown in Table 2, and mixed with a high-shear granulator for 10 minutes to obtain a mixed powder. The mixed powder was compacted using a roller compactor (TF-mini, manufactured by FREUND CORPORATION) at a pressure of 7 MPa and a roll rotation speed of 2.5 revolutions per minute, and then crushed using a Co-mil (U-5, manufactured by Quadro) with a screen with a hole diameter of 0.991 mm to obtain granules. Using a tablet press (Virgo01, manufactured by Kikusui Seisakusho), 420 mg of the granules were filled into a die with a major diameter of 14 mm and a minor diameter of 6.5 mm and compacted at a pressure of 19 kN to obtain uncoated tablets.

Using a coating machine (HIGH-COATER lab, manufactured by FREUND CORPORATION), approximately 21 mg of the film-coating agent was film-coated on each tablet to obtain film-coated tablets.

### (Example 20)

Uncoated tablet components were weighed in the ratios shown in Table 2, and uncoated tablets were obtained using the method according to Example 12. A screen with a hole diameter of 2 mm was used for the Power Mill.

The film-coating components were weighed in the ratios shown in Table 2, and dissolved and suspended in water to obtain a film-coating solution. Using a coating machine (HIGH-COATER lab, manufactured by FREUND CORPORATION), approximately 20 mg of the film-coating agent was film-coated on each tablet to obtain film-coated tablets.

**[Table 2]**

| Component | Blending Amount (mg/tab) | | | | | | | |
|---|---|---|---|---|---|---|---|---|
| | Example 13 | Example 14 | Example 15 | Example 16 | Example 17 | Example 18 | Example 19 | Example 20 |
| Uncoated Tablet Component | | | | | | | | |
| Valemetostat Tosylate (As Valemetostat) | 135.3 (100) | 135.3 (100) | 135.3 (100) | 135.3 (100) | 135.3 (100) | 135.3 (100) | 135.3 (100) | 135.3 (100) |
| Microcrystalline Cellulose (Ceolus PH-301, Asahi Kasei) | 167.1 | 128.7 | 188.1 | 167.1^{a} | 167.1 | 167.1 | 167.1 | 167.1 |
| Croscarmellose Sodium (Primellose, DFE Pharma) | 63 | 31.5 | - | 63 | 63 | 63 | 63 | 63 |
| Sodium Starch Glycolate (Explotab, JRS Pharma) | 42 | - | 84 | 42 | 42 | 42 | 42 | 42 |
| Light Anhydrous Silicic Acid (Aerosil 200, NIPPON AEROSIL) | - | 7 | - | - | - | - | - | - |
| Light Anhydrous Silicic Acid (SYLYSIA 320TP, FUJI SILYSIA CHEMICAL) | 6.3 | - | 6.3 | 6.3 | 6.3 | 6.3 | 6.3 | 6.3 |
| Sodium Stearyl Fumarate (Pruv, JRS Pharma) | - | 17.5 | - | - | - | - | - | - |
| Magnesium Stearate (Spec Gx) | 6.3 | - | 6.3 | 6.3 | 6.3 | 6.3 | 6.3 | 6.3 |
| Uncoated Tablet Total | 420 | 320 | 420 | 420 | 420 | 420 | 420 | 420 |

| Film-Coating Component | | | | | | | | |
|---|---|---|---|---|---|---|---|---|
| Pre-mix Film-Coating Agent OPADRY (Colorcon Japan) | 20 | 10 | 20 | - | - | - | - | - |
| Pre-mix Film-Coating Agent OPADRYAMB (Colorcon Japan) | - | - | - | - | - | 21 | - | - |
| Pre-mix Film-Coating Agent OPADRYII (Colorcon Japan) | - | - | - | - | - | - | 21 | - |
| Pre-mix Film-Coating Agent OPADRY300 (Colorcon Japan) | - | - | - | - | - | - | - | 20 |
| Hypromellose (6 cp) (TC-5 R, Shin-Etsu Chemical) | - | - | - | 18.7 | - | - | - | - |
| Hypromellose (15 cp) (TC-5 S, Shin-Etsu Chemical) | | | | - | 16.4 | | | |
| Titanium Dioxide (A-HR, FREUND CORPORATION) | | | | 2.7 | 3.2 | | | |
| Talc (Matsumura Sangyo) | | | | 2.7 | 3.2 | | | |
| Macrogol 400 (Sanyokasei) | | | | - | 1.2 | | | |
| PVP/VA^{b} (S-630, Ashland) | | | | 2.0 | - | | | |
| Film-Coating Agent Total | 20 | 10 | 20 | 26 | 24 | 21 | 21 | 20 |
| Coverage (%)^{c} | 5 | 3 | 5 | 6 | 6 | 5 | 5 | 5 |
| Film-Coated Tablet Total | 440 | 330 | 440 | 446 | 444 | 441 | 441 | 440 |

| | | | | | | | | |
|---|---|---|---|---|---|---|---|---|
| "-" indicates that the component is not blended a: In Example 16 only, Ceolus PH-302 manufactured by Asahi Kasei was used b: Vinyl Acetate/Vinylpyrrolidone Copolymer c: Percentage of film coating agent mass divided by uncoated tablet mass | | | | | | | | |

### (Example 21)

Uncoated tablet components were weighed in the ratios shown in Table 3, and mixed with a high-shear granulator to obtain a mixed powder. The mixed powder was compacted using a roller compactor (FP90x30, manufactured by FREUND CORPORATION) at a roll pressure of 17.5 MPa and a roll rotation speed of 8 revolutions per minute, and then crushed using the screen provided with the roller compactor to obtain granules. Using a tablet press (AQUC, manufactured by Kikusui Seisakusho), 420 mg of the granules were filled into a die with a major diameter of 14 mm and a minor diameter of 6.5 mm and compacted at a pressure of 21 kN to obtain uncoated tablets.

Using a coating machine (PRC-GTX mini, manufactured by Powrex), approximately 2 mg of the film-coating agent was film-coated on each tablet to obtain film-coated tablets.

### (Example 22)

Uncoated tablet components were weighed in the ratios shown in Table 3, and uncoated tablets were obtained using the method according to Example 12. However, a screen with a hole diameter of 2 mm was used for the Power Mill.

The film-coating components were weighed in the ratios shown in Table 3, and dissolved and suspended in water to obtain a film-coating solution. Using a coating machine (HIGH-COATER lab, manufactured by FREUND CORPORATION), approximately 16 mg of the film-coating agent was film-coated on each tablet to obtain film-coated tablets.

### (Example 23)

Film coating was performed in the same manner using the uncoated tablets and film-coating solution of Example 22. Approximately 28 mg of the film-coating agent was film-coated on each tablet to obtain film-coated tablets.

**[Table 3]**

| Component | Blending Ratio (mg/tab) | | |
|---|---|---|---|
| | Example 21 | Example 22 | Example 23 |
| Uncoated Tablet Component | | | |
| Valemetostat Tosylate (As Valemetostat) | 135.3 (100) | 135.3 (100) | 135.3 (100) |
| Microcrystalline Cellulose (Ceolus PH-301 , Asahi Kasei) | 167.1 | - | - |
| Microcrystalline Cellulose(Ceolus PH-302, Asahi Kasei) | - | 167.1 | 167.1 |
| Croscarmellose Sodium (Primellose, DFE Pharma) | 63 | 63 | 63 |
| Sodium Starch Glycolate (Explotab, JRS Pharma) | 42 | 42 | 42 |
| Light Anhydrous Silicic Acid (SYLYSIA 320TP, FUJI SILYSIA CHEMICAL) | 6.3 | 6.3 | 6.3 |
| Magnesium Stearate (Spec Gx) | 6.3 | 6.3 | 6.3 |
| Uncoated Tablet Total | 420 | 420 | 420 |

| Film-Coating Component | | | |
|---|---|---|---|
| Pre-mix Film-Coating Agent OPADRY (Colorcon Japan) | 2 | - | - |
| Hypromellose (6 cp) (TC-5 R, Shin-Etsu Chemical) | - | 9.7 | 17.0 |
| Titanium Dioxide (A-HR, FREUND CORPORATION) | - | 2.8 | 4.9 |
| Talc (Matsumura Sangyo) | - | 1.6 | 2.8 |
| Macrogol 6000 (NOF CORPORATION) | - | 1.9 | 3.4 |
| Film-Coating Agent Total | 2 | 16 | 28 |
| Coverage (%)^{a} | 0.5 | 4 | 7 |
| Film-Coated Tablet Total | 422 | 436 | 448 |

| | | | |
|---|---|---|---|
| "-" indicates that the component is not blended a: Percentage of film coating agent mass divided by uncoated tablet mass | | | |

### (Example 24)

Uncoated tablet components were weighed in the ratios shown in Table 4, and granules were obtained using the method according to Example 19. 105 mg of the granules were filled into a die with a diameter of 6.5 mm and compacted at a pressure of 9 kN to obtain uncoated tablets.

Using a coating machine (HIGH-COATER lab, manufactured by FREUND CORPORATION), approximately 5 mg of the film-coating agent was film-coated on each tablet to obtain film-coated tablets.

### (Example 25)

Uncoated tablet components were weighed in the ratios shown in Table 4, and granules were obtained using the method according to Example 21. However, they were produced under the conditions of a roll pressure of 19 MPa and a roll rotation speed of 11.5 revolutions per minute. 210 mg of the granules were filled into a die with a diameter of 8 mm and compressed at a tableting pressure of 13 kN to obtain uncoated tablets.

Using a coating machine (PRC-GTX mini, manufactured by Powrex), approximately 10 mg of the film-coating agent was film-coated on each tablet to obtain film-coated tablets.

### (Example 26)

Uncoated tablet components were weighed in the ratios shown in Table 4, and granules were obtained using the method according to Comparative Example 6. 462 mg of the granules were filled into a die with a major diameter of 14 mm and a minor diameter of 6.5 mm and compacted at a pressure of 15 kN to obtain uncoated tablets.

**[Table 4]**

| Component | Example 24 | | Example 25 | | Example 14 | | Example 26 | |
|---|---|---|---|---|---|---|---|---|
| | Mass (mg) | Ratio (%) | Mass (mg) | Ratio (%) | Mass (mg) | Ratio (%) | Mass (mg) | Ratio (%) |
| Valemetostat Tosylate (As Valemetostat) | 33.82 (25) | 32 (24) | 67.64 (50) | 32 (24) | 135.3 (100) | 42 (31) | 270.6 (200) | 59 (43) |
| Microcrystalline Cellulose (Ceolus PH-301, Asahi Kasei) | 41.78 | 40 | 83.56 | 40 | 128.7 | 40 | 90.2 | 20 |
| Croscarmellose Sodium (Primellose, DFE Pharma) | 15.75 | 15 | 31.5 | 15 | 31.5 | 10 | 66 | 14 |
| Sodium Starch Glycolate (Explotab, JRS Pharma) | 10.5 | 10 | 21 | 10 | - | - | - | - |
| Light Anhydrous Silicic Acid (Aerosil 200, NIPPON AEROSIL) | - | - | - | - | 7 | 2.2 | 10.06 | 2.2 |
| Light Anhydrous Silicic Acid (SYLYSIA 320TP, FUJI SILYSIA CHEMICAL) | 1.575 | 1.5 | 3.15 | 1.5 | - | - | - | - |
| Sodium Stearyl Fumarate (Pruv, JRS Pharma) | - | - | - | - | 17.5 | 5.5 | 25.16 | 5.4 |
| Magnesium Stearate (Spec Gx) | 1.575 | 1.5 | 3.15 | 1.5 | - | - | - | - |
| Uncoated Tablet Total | 105 | 100 | 210 | 100 | 320 | 100 | 462 | 100 |
| Pre-mix Film-Coating Agent OPADRY (Colorcon Japan) | 5 | - | 10 | - | 10 | - | - | - |
| Film-Coated Tablet Total | 110 | - | 220 | - | 330 | - | - | - |

| | | | | | | | | |
|---|---|---|---|---|---|---|---|---|
| "-" indicates that the component is not blended | | | | | | | | |

### (Test Example 1) Dissolution Test Using Uncoated Tablets

The die of Comparative Example 1 was fixed to a shaft using the Basket Method described in the Japanese Pharmacopoeia. For the dissolution test, the apparatus described in the Dissolution Test Method of the Japanese Pharmacopoeia was used. The upper part of the powder surface of the compacted product was prevented from being in contact with the test solution, while the lower part of the powder surface was allowed to be in contact with the test solution. After setting the lower part of the powder surface to be approximately 2 cm from the bottom of the vessel, 900 mL of the second fluid for the Dissolution Test in the Japanese Pharmacopoeia was added, and while rotating the die containing compacted product at 50 rotations per minute, the dissolution amount of valemetostat from the compacted product was determined from the absorbance of the test solution by UV spectroscopy (measurement wavelength: 295 nm, control: 360 nm) (for the method for evaluating the drug dissolution rate from the powder surface, the method described in Tsukinaka Yamana's, "Iyakuhin Sokudoron (Pharmaceutical Kinetics)" was referred to).

For the tablets described in Comparative Examples 2 to 6 and Examples 1 to 12, a dissolution test at 50 rotations per minute was performed in 900 mL of the second fluid for the Dissolution Test in the Japanese Pharmacopoeia by the Paddle Method described in the Dissolution Test Method of the Japanese Pharmacopoeia and the absorbance of the test solution was measured by UV spectroscopy (measurement wavelength: 295 nm and reference: 360 nm, or measurement wavelength: 297 nm without reference subtraction) to measure the dissolution rate.

The results are shown in Table 5.

The tablets of Comparative Examples 1 to 6 had very low dissolution rates of valemetostat tosylate. A translucent, poorly dissolved substance was produced at the surface of the tablets in contact with the test solution, and the inside of the tablets was dry, suggesting that the translucent, poorly dissolved substance prevented the dissolution of valemetostat tosylate. It was found that by blending croscarmellose sodium and/or sodium starch glycolate, the dissolution rate 60 minutes after starting the test of the tablets of Examples 1 to 12 greatly improved to 73 to 101%, without producing a poorly dissolved substance.

**[Table 5]**

| Comparative Example/Example No. | Dissolution Rate 60 Minutes after Starting Test (Percentage to the labeled amount) |
|---|---|
| Comparative Example 1 | -^{a} |
| Comparative Example 2 | 4 |
| Comparative Example 3 | 5 |
| Comparative Example 4 | 6 |
| Comparative Example 5 | 4 |
| Comparative Example 6 | 15 |
| Example 1 | 73 |
| Example 2 | 87 |
| Example 3 | 95 |
| Example 4 | 95 |
| Example 5 | 92 |
| Example 6 | 101 |
| Example 7 | 96 |
| Example 8 | 98 |
| Example 9 | 94 |
| Example 10 | 92 |
| Example 11 | 98 |
| Example 12 | 97 |

| | |
|---|---|
| a: 2.5 mg was dissolved. | |

(Test Example 2) Stability and Dissolution Test Using Film-Coated Tablets 1

The uncoated tablets and film-coated tablets described in Example 13 were placed in plastic bottles (DUMA TWIST-OFF, manufactured by Gerresheimer, capacity: 50 mL), used as stability test specimens, and stored in a stability chamber at 60°C for one week. The pharmaceutical compositions of Example 13 were each packaged in PTP and used as stability test specimens.

The stability test specimens were stored in a stability chamber at 40°C/75%RH for 3 months. For each stability test specimen at the start of the stability test and after storage, a dissolution test at 50 rotations per minute was performed in 900 mL of the second fluid of the Dissolution Test in the Japanese Pharmacopoeia by the Paddle Method described in the Dissolution Test Method of the Japanese Pharmacopoeia, and the absorbance of the test solution was measured by UV spectroscopy (measurement wavelength: 297 nm) to measure the dissolution rate. The results are shown in Table 6.

The film-coated tablets (FC tablets) described in Example 13 showed better quality even after storage compared to the uncoated tablets.

**[Table 6]**

| Example No. | | Example 13 | |
|---|---|---|---|
| Dosage form | | Uncoated Tablet | FC Tablet |
| Dissolution Rate (60 Minutes after Starting Test) | At Start of Stability Test | 96 | 96 |
| | 1 week at 60°C | 51 | 87 |
| | 3 months at 40°C/75%RH | - | 88 |

### (Test Example 3) Stability Test and Dissolution Test Using Film-Coated Tablets 2

The uncoated tablets and film-coated tablets described in Examples 14, 15, 17 and 20 were each placed in plastic bottles (DUMA TWIST-OFF, manufactured by Gerresheimer, capacity: 50 mL) and used as stability test specimens. After packaging the film-coated tablets of Example 16 in PTP, the PTP sheets were sealed in aluminum bags and used as stability test specimens. The film-coated tablets of Examples 18 and 19 were placed in plastic bottles (TCB, No. 4, manufactured by Taisei Kako) and used as stability test specimens. Each stability test specimen was stored in a stability chamber at 60°C for one week, in a stability chamber at 40°C/75%RH for 4 weeks, 3 months, or 6 months. For the specimen at the start of the stability test and each specimen after storage, a dissolution test at 50 rotations per minute was applied in 900 mL of the second fluid of the Dissolution Test in the Japanese Pharmacopoeia by the Paddle Method described in the Dissolution Test Method of the Japanese Pharmacopoeia, and the absorbance was measured by UV spectroscopy (measurement wavelength: 295 nm, reference: 360 nm, or measurement wavelength: 297 nm without reference subtraction) to measure the dissolution rate. The results are shown in Tables 7, 8 and 9.

As shown in Table 7 and Table 8, the film-coated tablets (FC tablets) described in Example 14 and Example 15 showed better dissolution properties compared to the uncoated tablets. As shown in Table 9, Examples 16 to 20 also showed good dissolution properties under all conditions.

**[Table 7]**

| Example No. | | Example 14 | |
|---|---|---|---|
| Dosage form | | Uncoated Tablet | FC Tablet |
| Dissolution Rate (60 Minutes after Starting Test) | At Start of Stability Test | 98 | 99 |
| | 1 week at 60°C | 62 | 83 |
| | 4 weeks at 40°C/75%RH | 83 | 95 |

**[Table 8]**

| Example No. | | Example 15 | |
|---|---|---|---|
| Dosage form | | Uncoated Tablet | FC Tablet |
| Dissolution Rate (60 Minutes after Starting Test) | At Start of Stability Test | 90 | 90 |
| | 1 week at 60°C | 59 | 92 |

**[Table 9]**

| Example No. | | Example 16 | Example 17 | Example 18 | Example 19 | Example 20 |
|---|---|---|---|---|---|---|
| Dosage form | | FC tablet | FC tablet | FC tablet | FC tablet | FC tablet |
| Film-Coating Agent | | TC-5R Titanium Dioxide Talc PVPVA | TC-5S Titanium Dioxide Talc Macrogol 400 | OpadryAMB | Opadry II | Opadry300 |
| Dissolution Rate (60 Minutes after Starting Test) | At Start of Stability Test | 94 | 95 | 98 | 98 | 95 |
| | 1 week at 60°C | 90 | 94 | 95 | 98 | 92 |
| | 1 month at 40°C/75%RH | 94 | - | 98 | 97 | - |
| | 3 months at 40°C/75%RH | 94 | - | - | - | - |
| | 6 months at 40°C/75%RH | - | - | 86 | - | - |

### (Test Example 4) Stability and Dissolution Test Using Film-Coated Tablets 3

The uncoated tablets and film-coated tablets of Example 21 were each placed in plastic bottles (DUMA TWIST-OFF, manufactured by Gerresheimer, capacity: 50 mL) and stored in a stability chamber at 60°C for 12 days. After packaging each film-coated tablet described in Examples 22 and 23 in PTP, the PTP sheets were sealed in aluminum bags, which were stored in a stability chamber at 60°C for 1 week. For the product at the start of the stability test and each product after storage, a dissolution test at 50 rotations per minute was performed in 900 mL of the second fluid of the Dissolution Test in the Japanese Pharmacopoeia by the Paddle Method described in the Dissolution Test Method of the Japanese Pharmacopoeia, and the absorbance at a wavelength of 297 nm was measured by UV spectroscopy to measure the dissolution rate. The results are shown in Table 10 and Table 11.

As shown in Table 10, improved storage stability was observed with film coating even at low coverage rates. In addition, as shown in Table 11, the stability of the film-coated tablets was similarly good at high coverage rates.

**[Table 10]**

| Example No. | | Example 21 | |
|---|---|---|---|
| Dosage form | | Uncoated Tablet | Film-Coated Tablet |
| Coverage (%) | | 0 | 0.5 |
| Dissolution Rate (60 Minutes after Starting Test) | At Start of Stability Test | 92 | 92 |
| | 12 days at 60°C | 52 | 81 |

**[Table 11]**

| Example No. | | Example 22 | Example 23 |
|---|---|---|---|
| Coverage (%) | | 4 | 7 |
| Dissolution Rate (60 Minutes after Starting Test) | At Start of Stability Test | 94 | 93 |
| | 1 week at 60°C | 94 | 94 |

### (Test Example 5) Dissolution Test at Various Contents and Blending Ratios

For the tablets described in Examples 24, 25, 14, and 26, a dissolution test at 50 rotations per minute was performed in 900 mL of the second fluid of the Dissolution Test in the Japanese Pharmacopoeia by the Paddle Method described in the Dissolution Test Method of the Japanese Pharmacopoeia. For Examples 24 and 25, the absorbance at a wavelength of 297 nm was measured by UV spectroscopy to measure the dissolution rate. For Examples 14 and 26, the absorbance of the test solution was measured by UV spectroscopy (measurement wavelength: 295 nm, reference: 360 nm) to measure the dissolution rate.
The results are shown in Table 12. All of the Examples showed good dissolution properties.

**[Table 12]**

| Example No. | Dissolution Rate 60 Minutes after Starting Test (Percentage to labeled amount) |
|---|---|
| Example 24 | 98 |
| Example 25 | 97 |
| Example 14 | 99 |
| Example 26 | 100 |

## Claims

1. A pharmaceutical composition comprising valemetostat or a pharmaceutically acceptable salt thereof, and one or more selected from croscarmellose sodium and sodium starch glycolate.

2. The pharmaceutical composition according to claim 1, further comprising a water-insoluble excipient.

3. The pharmaceutical composition according to claim 2, wherein the water-insoluble excipient is one or more selected from microcrystalline cellulose, partially pregelatinized starch, and pregelatinized starch.

4. The pharmaceutical composition according to claim 2 or 3, wherein the water-insoluble excipient is microcrystalline cellulose.

5. The pharmaceutical composition according to any one of claims 1 to 4, comprising 5 to 25% by mass of croscarmellose sodium relative to the total mass of the pharmaceutical composition.

6. The pharmaceutical composition according to any one of claims 1 to 5, comprising 5 to 45% by mass of sodium starch glycolate relative to the total mass of the pharmaceutical composition.

7. The pharmaceutical composition according to any one of claims 1 to 6, further comprising one or more lubricants.

8. The pharmaceutical composition according to any one of claims 1 to 7, which is a tablet.

9. The pharmaceutical composition according to claim 8, which is a film-coated tablet.

10. The pharmaceutical composition according to claim 9, wherein the film-coating component is one or more selected from hypromellose, polyvinyl alcohol, and polyvinyl alcohol/acrylic acid/methyl methacrylate copolymer.
